(19) Europäisches Patentamt
European Patent Office
Office européen des brevets

(11) EP 2 626 687 B1

(12) EUROPEAN PATENT SPECIFICATION

(45) Date of publication and mention
of the grant of the patent:
**06.03.2019 Bulletin 2019/10**

(21) Application number: **11830671.1**

(22) Date of filing: **04.10.2011**

(51) Int Cl.:
*G01N 19/00* (2006.01)    *G06F 17/50* (2006.01)

(86) International application number:
**PCT/JP2011/072886**

(87) International publication number:
**WO 2012/046739 (12.04.2012 Gazette 2012/15)**

(54) **METHOD FOR PREDICTING ELASTIC RESPONSE PERFORMANCE OF RUBBER PRODUCT, METHOD FOR DESIGN, AND DEVICE FOR PREDICTING ELASTIC RESPONSE PERFORMANCE**

VERFAHREN ZUR VORHERSAGE DES ELASTISCHEN ANSPRECHVERHALTENS EINES KAUTSCHUKPRODUKTS, DESIGNVERFAHREN DAFÜR UND VORRICHTUNG ZUR VORHERSAGE DES ELASTISCHEN ANSPRECHVERHALTENS EINES KAUTSCHUKPRODUKTS

PROCÉDÉ DE PRÉDICTION DES PERFORMANCES DE RÉPONSE ÉLASTIQUE D'UN ARTICLE EN CAOUTCHOUC, PROCÉDÉ DE CONCEPTION, ET DISPOSITIF DE PRÉDICTION DE PERFORMANCES DE RÉPONSE ÉLASTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.10.2010 JP 2010225984**

(43) Date of publication of application:
**14.08.2013 Bulletin 2013/33**

(73) Proprietor: **Bridgestone Corporation**
**Tokyo 104-8340 (JP)**

(72) Inventor: **AKUTAGAWA, Keizo**
**Tokyo 104-8340 (JP)**

(74) Representative: **Lamb, Martin John Carstairs**
**Marks & Clerk LLP**
**90 Long Acre**
**London WC2E 9RA (GB)**

(56) References cited:
**JP-A- 2007 272 416    JP-B2- 4 299 735**

• **ARRUDA ET AL: "A three-dimensional constitutive model for the large stretch behavior of rubber elastic materials", JOURNAL OF THE MECHANICS AND PHYSICS OF SOLIDS, vol. 41, no. 2, February 1993 (1993-02), pages 389-412, XP024446691, ISSN: 0022-5096, DOI: 10.1016/0022-5096(93)90013-6 [retrieved on 1993-02-01]**
• **YOSHIHISA MIYAMOTO: 'Ichijiku Shincho ni Okeru Polyisoprene Gomu no Kesshoka to Yukai' JOURNAL OF THE SOCIETY OF RUBBER INDUSTRY vol. 77, no. 1, 15 January 2004, pages 24 - 29, XP055123626**
• **TOMITA J.: 'Molecular chain network theory and its application to evaluation of mechanical characteristics of polymer' JOURNAL OF THE SOCIETY OF MATERIALS SCIENCE, JAPAN vol. 57, no. 3, 15 March 2008, pages 209 - 218, XP055083568**

**Description**

Technical Field

The present invention relates to an elastic response performance prediction method and design method for a rubber product, and to an elastic response performance prediction apparatus. In particular the present invention relates to a prediction method that employs a Finite Element Analysis (FEA) method to predict an elastic response performance of a rubber product, to a rubber product design method employing the prediction method, and to an elastic response performance prediction apparatus thereof.

Background Art

When designing a rubber product, methods that employ 3-D Finite Element Analysis (FEA) methods to predict the elastic response thereof, and that employ such analysis and simulation results have already been implemented for tens of years. Energy constitutive equations that are employed in FEA computation and in the analysis reflect a stress-strain relationship of a rubber material, migrate linear elasticity equations into Mooney-Rivlin equations, and recently non-linear constitutive equations are being introduced for regions of large deformation in such energy constitutive equations.

Moreover, recently with regard to such a rubber material constitutive equation, under the principles of lattice deformation theory developed with molecular statistical thermodynamics based on the extension of rubber molecular chains, there is a proposal for a constitutive equation that is capable of expressing temperature dependence, an important factor in the design of a lot of industrial rubber materials, such as tires, using a parameter with a physical meaning (Japanese Patent Publication 4299735).

A summary of such a constitutive equation follows.

First, tensional stress in rubber is expressed by a shear elastic modulus G and a tension direction extension ratio $\lambda$ using the following Equation (1).

$$\sigma = 2G\left(\lambda - \frac{1}{\lambda^2}\right) \qquad \cdots (1)$$

Moreover, the shear elastic modulus G of rubber is computable, as shown in the following Equation (2), by differentiating the Helmholtz free energy A of statistical thermodynamics with respect to $I_1$.

$$\frac{G}{2} = \frac{\partial A}{\partial I_1} \qquad \cdots (2)$$

The average energy $\overline{A}_\alpha$ of a system in a thermodynamic equilibrium state is expressed by the following Equation (3).

$$\overline{A}_\alpha = \frac{1}{Z}\sum A_\alpha e^{-\beta A_\alpha} = -\left(\frac{\partial \log Z}{\partial \beta}\right)_{\{r\}} \qquad \cdots (3)$$

$\beta$ here is equivalent to $1/(k\Delta T)$, wherein k represents the Helmholtz constant and $\Delta T$ represents the difference from a polymer glass transition temperature $T_g$ to the measured temperature T. Moreover, $A_\alpha$ represents the energy level. Z is a distribution function for standardizing the energy of the system, and is expressed by the following Equation (4).

$$Z = \sum_\alpha e^{-\beta A_\alpha} = \sum_\alpha e^{-\beta(U_\alpha - TS_\alpha)} \qquad \cdots (4)$$

Here, as expressed in the following Equation (5), $U_\alpha$ can be assumed to be equivalent to Hamiltonian H from a statistical thermodynamic perspective.

$$U_\alpha = H \,(\text{Temp, Constraints}) \qquad\qquad \ldots (5)$$

Wherein, the Hamiltonian H is expressed as a function of temperature conditions and constraint conditions defining microstates in statistical thermodynamics.

Two states are set, a low temperature state and a high temperature state, as a model expressing temperature dependence of rubber molecules, and state numbers of rubber molecule called $r_1$ and $r_2$ are distributed in strain energy fields called $I_{1a}$ and $I_{1b}$. This can be represented by the following Equation (6) when this is applied to express all of the states as a function.

$$Z = \sum_\alpha e^{-\beta A_\alpha} = \sum_\alpha e^{-\beta(U_\alpha - TS_\alpha)} = e^{\beta TS}\left( e^{-\frac{-I_{1a}-I_{1b}-\kappa}{k\Delta T}} + e^{-\frac{-I_{1a}+I_{1b}}{k\Delta T}} + e^{\frac{I_{1a}-I_{1b}}{k\Delta T}} + e^{-\frac{I_{1b}+I_{1b}-\kappa}{k\Delta T}} \right)$$

$$= 2e^{\beta TS}\left[ e^{\frac{\kappa}{k\Delta T}} \cosh\left(\frac{I_{1a}+I_{1b}}{k\Delta T}\right) + \cosh\left(\frac{I_{1a}-I_{1b}}{k\Delta T}\right) \right] \qquad \cdots (6)$$

Wherein $\kappa$ represents molecular recoil energy. Two states of different rubber molecule energy states are represented by $r_1 = -1$, $r_2 = 1$, and the statistical thermodynamic average thereof is represented by $<r_1 \cdot r_2>$. This product expresses the two extreme end energy states, and when this product is +1 this represents rubber molecules being in the same state, and when this product is -1 this represents rubber molecules being in different states. Assuming that the strain energy field contribution is equal for all of the molecules, then by making $I_1 = 1_{1a} = I_{1b}$, Equation (6) above can be expressed by the following Equation (7).

$$Z = \sum_\alpha e^{-\beta A_\alpha} = 2e^{\beta TS}\left[ e^{\frac{\kappa}{k\Delta T}} \cosh(2\beta \cdot I_1) + 1 \right] \qquad \cdots (7)$$

Substituting Equation (7) in above Equation (3) expresses the rubber elastic modulus G by the following Equation (9).

$$\bar{A}_\alpha = \frac{1}{Z}\sum A_\alpha e^{-\beta A_\alpha} = -\left(\frac{\partial \log Z}{\partial \beta}\right)_{\{r\}} = -\frac{\partial}{\partial \beta}\left[ e^{\frac{\kappa}{k\Delta T}} \cosh(2\beta \cdot I_1) + 1 \right] - T \cdot S \qquad \cdots (8)$$

$$\frac{G}{2} = \frac{\partial \bar{A}_\alpha}{\partial I_1} = \frac{\partial U}{\partial I_1} - T \cdot \frac{\partial S}{\partial I_1} = -\frac{\partial}{\partial \beta \partial I_1}\left[ e^{\frac{\kappa}{k\Delta T}} \cosh(2\beta \cdot I_1) + 1 \right] - T \cdot \frac{\partial S}{\partial I_1} \qquad \cdots (9)$$

Considering a stretched molecule, the second term of the entropy term in Equation (9) is expressed using a Langevin function by the following Equation (10).

$$T \cdot \frac{\partial S}{\partial I_1} = -vRT\left[ \frac{1}{2} + \frac{3}{50n}\left(3I_1 - \frac{2}{\lambda}\right) + \frac{297}{6125n^2}\left(5I_1^2 - 4I_2 - 4\frac{I_1}{\lambda}\right) \right] \qquad \cdots (10)$$

Wherein n is defined as the number of links of a statistical molecule chain between cross-linked points.

Accordingly, Japanese Patent No. 4299735 proposes a constitutive equation that is capable of representing temperature dependence and employs a parameter with physical meaning.

Moreover, as illustrated in Fig. 6, a strain energy function (solid line) disclosed in Japanese Patent No. 4299735 is able to replicate test values (plots) over a wide range of extension ratios and temperature regions. Attention is also drawn to the disclosure of E. Arruda et al.: "A three-dimensional constitutive model for the large stretch behavior of rubber elastic materials", Journal of the Mechanics and Physics of Solids 41, no. 2, pages 389-412 (1993) and to JP2007-272416 which disclose a method for predicting the elastic response performance of a rubber product by using a constitutive equation indicating temperature and strain dependency of the rubber material using a statistical molecule chain model but do not include extension crystallization. The effects of crystallization of rubber under uniaxial deformation are studied by Y. Miyamoto in "Crystallization and Melting of Polyisoprene Rubber under Uniaxial Deformation", Journal of the Society of Rubber Industry, vol. 77, n. 1, p. 24-29 (2004) but this phenomenon is not incorporated in a statistical molecule chain model.

DISCLOSURE OF INVENTION

Technical Problem

However, in the strain energy function disclosed in Japanese Patent Publication 4299735, since consideration is not given to extension crystallization, this leads to the issue that the applicable range thereof is limited to rubbers without extension crystallization properties, such as styrene butadiene rubber.

The present invention is directed towards solving the above issue, and an object thereof is to provide an elastic response performance prediction method, a rubber product design method and an elastic response performance prediction apparatus that are capable of raising prediction precision of the elastic response performance of rubber products even when the rubber product has extension crystallization properties.

Solution to Problem

In order to achieve the above objective, a first aspect of the present invention is an elastic response performance prediction method as claimed in claim 1.

A second aspect of the present invention is an elastic response performance prediction apparatus as claimed in claim 7.

According to the first aspect and the second aspect of the present invention, when a filler based rubber that exhibits extension crystallization properties is observed at the micro-observation level, in a diffuse deformation region of a rubber product, a constitutive equation is employed that expresses temperature and strain dependence of strain energy, and that incorporates the number of links between the cross-linked points in the statistical molecule chain which is expressed using the parameter representing extension crystallization. Accordingly, elastic response performance of a rubber product can be predicted with good precision even when the rubber product exhibits extension crystallization properties.

According to a third aspect of the present invention, the crystallization ratio $X_c$ may be set at 0 when the material of the rubber product does not exhibit extension crystallization properties.

According to a fourth aspect of the present invention, the constitutive equation is the following Equation (III):

$$\Delta A = \left(U_1 - TS_1\right) + p\left(V_1 - V_0\right) - \left(U_0 - TS_0\right) \qquad \cdots \text{ (III)}$$

Wherein A represents Helmholtz free energy, $U_0$ represents internal energy in a non-deformed state and $U_1$ represents internal energy in a deformed state. p represents pressure, $V_0$ represents volume in a non-deformed state and $V_1$ represents volume in a deformed state. T represents absolute temperature, $S_0$ represents entropy in a non-deformed state, and $S_1$ represents entropy in a deformed state. Each of the terms of Equation (III) are expressed by the following Equations (IV) to (VI):

$$U_1 - T \cdot S_1 = \frac{e^{\beta' \cdot \kappa}\left\{\kappa \cosh\left(2\beta'\left(I_1'-3\right)\right) + 2\left(I_1'-3\right) \cdot \sinh\left(2\beta'\left(I_1'-3\right)\right)\right\}}{e^{\beta' \cdot \kappa} \cosh\left(2\beta'\left(I_1'-3\right)\right) + 1}$$
$$- \frac{N}{\beta} \cdot \left\{\frac{1}{2}I_1 + \frac{3}{100n}\left(3I_1^2 - 4I_2\right) + \frac{99}{12250n}\left(5I_1^3 - 12I_1I_2\right)\right\}$$
$$\cdots \quad \text{(IV)}$$

$$p(V_1 - V_0) = B \cdot (V_1 - V_0) = B\left(I_3^{\frac{1}{2}} - 1\right)^2$$

$$- \frac{1}{\beta'}\left\{\ln\left[1 + e^{\beta' \cdot \kappa} \cosh(2\beta'(I_1' - 3))\right] - \ln\left[1 + e^{\beta' \cdot \kappa}\right]\right\}$$

$$\cdots \quad (V)$$

$$U_0 - T \cdot S_0 = \frac{\kappa \cdot e^{\beta' \cdot \kappa}}{e^{\beta' \cdot \kappa} + 1} + \frac{N}{\beta}\left(\frac{3}{2} + \frac{45}{100n} + \frac{2673}{12250n^2}\right) \quad \cdots \quad (VI)$$

Wherein: $I_1$, $I_2$, and $I_3$ are expressed as functions of three extension ratios of deformation $\lambda_1$, $\lambda_2$ and $X_3$ in xyz directions in three dimensional axes of rubber by $I_1 = \lambda_1^2 + \lambda_2^2 + \lambda_3^2$, $I_2 = \lambda_1^2 \cdot \lambda_2^2 + \lambda_2^2 \cdot \lambda_3^2 + \lambda_3^2 \cdot \lambda_1^2$, and $I_3 = \lambda_1^2 \cdot \lambda_2^2 \cdot \lambda_3^2$. n represents a number of links between the cross-linked points in the statistical molecule chain and $\kappa$ expresses an intermolecular interaction energy coefficient. $\beta = 1/RT$ and $\beta' = 1/R$ ($T - T_g$) wherein R is a gas constant and $T_g$ is a glass transition temperature, B represents bulk modulus, N represents number of molecules, and $I_1'$ is expressed using a local interaction function $\lambda_{micro}$ as a parameter expressing the intermolecular interaction by the following Equation (VII):

$$I_1' = \lambda_{micro}^2\left(\lambda_1^2 + \lambda_2^2 + \lambda_3^2\right) = \lambda_{micro}^2 \cdot I_1 \quad \cdots \quad (VII)$$

According to the first aspect and the second aspect of the present invention, when a filler based rubber that exhibits extension crystallization properties is observed at the micro-observation level, in a diffuse deformation region of a rubber product, a constitutive equation is employed that expresses temperature and strain dependence of an elastic modulus, and that incorporates the number of links between the cross-linked points in the statistical molecule chain which is expressed using the parameter representing extension crystallization. Accordingly, elastic response performance of a rubber product can be predicted with good precision even when the rubber product exhibits extension crystallization properties.

In a fifth aspect of the present invention the constitutive equation is the following Equation (X):

$$G = \frac{\partial W}{\partial I_1} = \frac{\partial A}{\partial I_1} = \frac{\partial U}{\partial I_1} - T \cdot \frac{\partial S}{\partial I_1} + \frac{\partial pV}{\partial I_1} \quad \cdots \quad (X)$$

Wherein G represents a shear elastic modulus, W represents a strain energy coefficient and A represents Helmholtz free energy. U represents internal energy, T represents absolute temperature and S represents entropy. $I_1$ is expressed as a function of three extension ratios of deformation $\lambda_1$, $\lambda_2$ and $\lambda_3$ in xyz directions in three dimensional axes of rubber by $I_1 = \lambda_1^2 + \lambda_2^2 + \lambda_3^2$. Each of the terms of Equation (X) is respectively expressed by the following Equation (XI), Equation (XII), and Equation (XIII):

$$\frac{\partial U}{\partial I_1}$$
$$= \frac{-e^{\beta'\kappa}\left\{2e^{\beta'\kappa}\sinh(2\beta'(I_1'-3))\cosh(2\beta'(I_1'-3)) + 2(\beta'\kappa+1)\sinh(2\beta'(I_1'-3)) + 4\beta'(I_1'-3)\cosh(2\beta'(I_1'-3)) + 4\beta'(I_1'-3)e^{\beta'\kappa}\right\}}{\left\{e^{\beta'\kappa} \cdot \cosh(2\beta'(I_1'-3)) + 1\right\}^2}$$

$$\cdots \quad (XI)$$

$$\frac{\partial S}{\partial I_1} = -vR\left[\frac{1}{2} + \frac{3}{50n}\left(3I_1 - \frac{2}{\lambda}\right) + \frac{297}{6125n^2}\left(5I_1^2 - 4I_2 - 4\frac{I_1}{\lambda}\right)\right] \quad \cdots \quad (XII)$$

$$\frac{\partial pV}{\partial I_1} = \frac{2 \cdot e^{\beta'\kappa} \cdot \cosh(2\beta'(I_1'-3))}{e^{\beta'\kappa} \cdot \cosh(2\beta'(I_1'-3))+1} \qquad \cdot \cdot \cdot \quad (\mathrm{X\,III})$$

Wherein n represents a number of links between the cross-linked points in the statistical molecule chain, $\kappa$ expresses an intermolecular interaction energy coefficient, v represents a cross-link density and $\lambda$ represents an extension ratio or compression ratio. $\beta' = 1/R\,(T - T_g)$ wherein R is a gas constant and $T_g$ is a glass transition temperature. P represents pressure and V represents volume. The term n is also a function of AU through $X_c$, and so needs to be differentiated with respect to $I_1$, however since the contribution is small the integrated term is ignored. $I_2$ is represented by $I_2 = \lambda_1^2 \cdot \lambda_2^2 + \lambda_2^2 \cdot \lambda_3^2 + \lambda_3^2 \cdot \lambda_1^2$. $I_1'$ is expressed using a local interaction function $\lambda_{micro}$ as a parameter expressing the intermolecular interaction by following Equation (XIV):

$$I_1' = \lambda_{micro}{}^2\left(\lambda_1{}^2 + \lambda_2{}^2 + \lambda_3{}^2\right) = \lambda_{micro}{}^2 \cdot I_1 \qquad \cdot \cdot \cdot \quad (\mathrm{X\,IV})$$

According to a sixth aspect of the present invention, the elastic response performance expressing deformation behavior of the rubber product may be predicted using a finite element analysis method.

In the present invention as described in detail above, the elastic response performance of a rubber product is predicted employing a constitutive equation that expresses temperature and strain dependence of strain energy in a rubber product, or by employing a constitutive equation that expresses temperature and strain dependence of an elastic modulus of the rubber product. It is accordingly possible through necessary simulation, and in particular through simulation at the micro-level of rubber, to design an optimum rubber material at the micro-level to achieve desired characteristics of a rubber product. A design method for rubber products that has both good efficiency and precision can also be provided. Advantageous Effects of Invention

As explained above, according to the elastic response performance prediction method, the rubber product design method and the elastic response performance prediction apparatus of the present invention, an elastic response performance of a rubber product is predicted by employing a constitutive equation that expresses temperature and strain dependence of strain energy in the rubber product, and that incorporates a number of links between cross-linked points in a statistical molecule chain which is expressed using a parameter representing extension crystallization, or by employing a constitutive equation that expresses temperature and strain dependence of an elastic modulus, and that incorporates a number of links between cross-linked points in a statistical molecule chain which is expressed using a parameter representing extension crystallization. An advantageous effect is accordingly achieved of enabling the elastic response performance of the rubber product to be predicted with good precision even when the rubber product exhibits extension crystallization properties.

BRIEF DESCRIPTION OF DRAWINGS

Fig. 1 is a schematic diagram illustrating an elastic response performance prediction apparatus according to an exemplary embodiment of the present invention.
Fig. 2 is an image of a microstructure model.
Fig. 3 is a graph illustrating prediction values and observed values related to the temperature dependence of a stress-strain curve of cross-linked styrene butadiene rubber.
Fig. 4 is a graph illustrating measurement values and computed values related to stress due to energy elasticity and stress due to entropy elasticity of a stress-strain curve at room temperature for a cross-linked styrene butadiene rubber.
Fig. 5 is a graph illustrating measurement values and computed values related to stress due to energy elasticity and stress due to entropy elasticity of a stress-strain curve at room temperature for a cross-linked natural rubber.

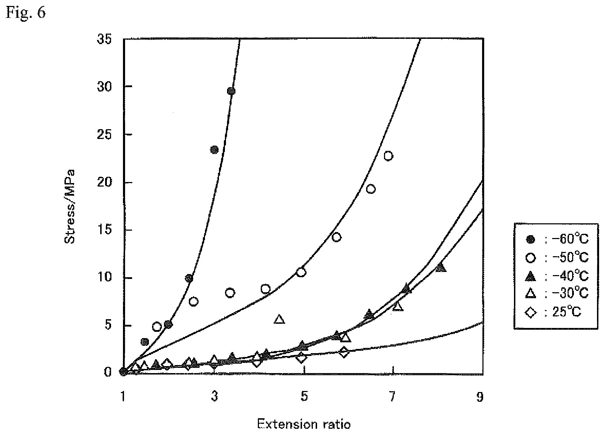

Fig. 6 is a graph illustrating prediction values related to temperature dependence of a stress-strain curve for rubber using related technology and observed values.

BEST MODE FOR CARRYING OUT THE INVENTION

Detailed explanation follows regarding an exemplary embodiment of the present invention, with reference to the drawings.

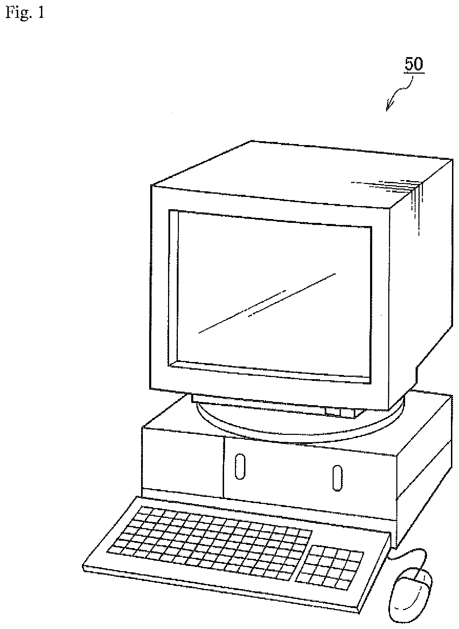

As illustrated in Fig. 1, an elastic response performance prediction apparatus 50 according to a first exemplary embodiment of the present invention is configured by a computer computation processing system that executes processing, described later, with an elastic response performance prediction program in order to execute elastic response performance prediction. Note that such a computer system is, for example, configured with a CPU, ROM, RAM, a hard disk, an input-output terminal, and other required units. The elastic response performance prediction program referred to above is preinstalled on a hard disk or the like.

The elastic response performance prediction method of the first exemplary embodiment of the present invention employs a constitutive equation expressing temperature and strain dependence of strain energy in a rubber material constituting a rubber product, and in particular preferably employs the Equations (I) to (VII) described above, to predict the elastic response performance of the rubber product.

Explanation follows regarding derivation principles of a constitutive equation expressing the temperature and strain dependence of strain energy in a rubber material. In the following, a microstructure model is defined for statistical thermodynamic computation, and a constitutive equation is derived based on this model.

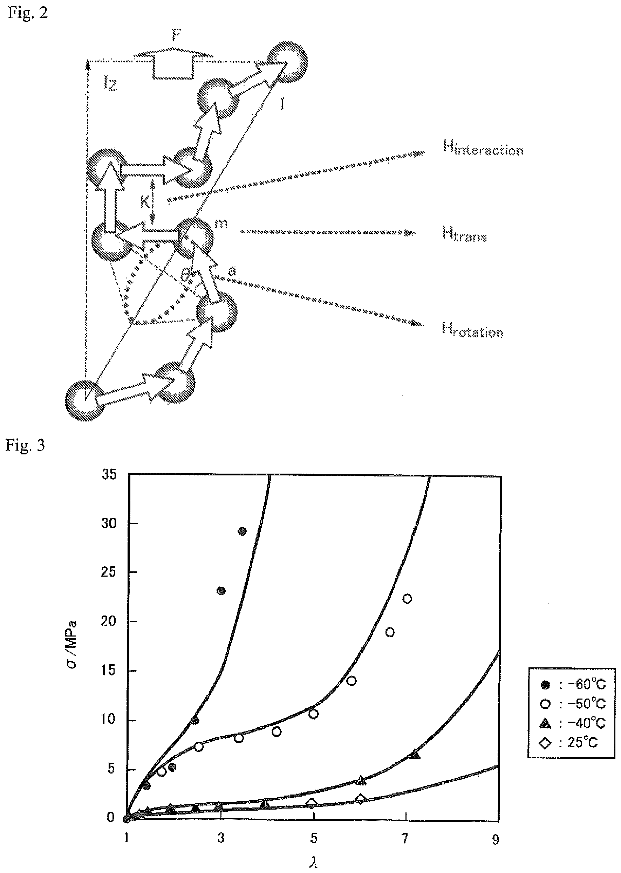

Fig. 2 illustrates a microstructure model. $H_{int}$ is a Hamiltonian expressing intermolecular or intramolecular interaction, $H_{rot}$ is a Hamiltonian expressing molecular rotation, and $H_{trans}$ is a Hamiltonian expressing molecular translation.

A thermodynamic equation representing this microstructure model can be expressed by the following Equation (11).

$$\Delta A = \Delta U + p\Delta V - T\Delta S = f\left(\overline{Z}_{int}, \overline{Z}_{rot}, \overline{Z}_{trans}\right) = g\left(H_{int}, H_{rot}, H_{trans}\right) \qquad \cdots (11)$$

Wherein: A represents Helmholtz free energy, U represents internal energy, and p represents pressure. V represents volume, T represents absolute temperature and S represents entropy. $\overline{Z}_{int}$ represents a distribution function expressing intermolecular or intramolecular interaction, $\overline{Z}_{rot}$ represents a distribution function expressing molecular rotation, and $\overline{Z}_{trans}$ represents a distribution function expressing molecular translation. The Hamiltonians corresponding to each of the molecular motion modes are expressed by the following Equation (12) to Equation (14).

$$H_{interaction} = \left(r_1 + r_2\right) \cdot I - \kappa \cdot \delta_{r_1, r_2} \qquad \cdots (12)$$

$$H_{trans} = \frac{p^2}{2 \cdot m} + \left(r_1 + r_2\right) \cdot I - \kappa \cdot \delta_{r_1, r_2} \qquad \cdots (13)$$

$$H_{rotation} = \frac{p_\theta^2}{2 \cdot I_M} + \frac{p_\phi^2}{2 \cdot I_M \cdot \sin^2 \theta} - F \cdot a \cdot \cos \theta \qquad \cdots (14)$$

Wherein $r_1$ and $r_2$ represent energy states of two molecular with intermolecular or intramolecular interaction. When $I_1'$ represents an invariant of intermolecular strain in the microstructure model illustrated in Fig. 2, then $r_1$ and $r_2$ are expressed by either +1 or -1. $r_1$ and $r_2$ corresponds to the four combinations of strain energy due to deformation represented by $I_1'$, (-1, -1), (+1, +1), (+1, -1), and (-1, +1). $\kappa$ is a coefficient expressing the magnitude of interaction energy. $\delta$ is a coefficient expressing a state involving interaction energy, and is -1 when two individual molecules are in the same energy state. When the two individual molecules are in the same energy state, $\kappa \cdot \delta$ becomes a minus coefficient, meaning stabilization by the amount of the interaction energy. However, when in different energy states, $\kappa \cdot \delta$ becomes 0, assuming that no interaction occurs. m expresses molecular mass, $P_\theta$ represents momentum expressing an angle of molecular rotation with respect to an axis connecting two molecules together. $P_\phi$ represents momentum expressing molecular rotation with respect to a rotation angle around the axis connecting two molecules together, P represents momentum with respect to a molecule of mass m, and $I_M$ an inertial moment of molecular rotation.

A relationship between a Hamiltonian and a distribution function is expressed by the following Equation (15).

$$z_\alpha = \sum \left\{ e^{\beta \cdot H_\alpha} \right\} \qquad \cdots \ (15)$$

Substituting Hamiltonians corresponding to each of the molecule motion modes into the relationship equation expressed above by Equation (15) gives each of the distribution functions expressed by the following Equation (16) to Equation (18).

$$z_{\text{int}} = \sum \left\{ e^{\beta'(-I_1'-I_1'-\kappa)} + e^{\beta'(-I_1'+I_1')} + e^{\beta'(I_1'-I_1')} + e^{\beta'(I_1'+I_1'-\kappa)} \right\} \qquad \cdots \ (16)$$

$$z_{rot} = \sum \exp\left\{ \beta \cdot \left( \frac{P_\theta^2}{2 \cdot I_M} + \frac{P_\varphi^2}{2 \cdot I_M \cdot \sin^2\theta} - F \cdot a \cdot \cos\theta \right) \right\} \qquad \cdots \ (17)$$

$$z_{trans} = \sum \exp\left\{ \beta \cdot \left( \frac{P^2}{2m} \right) \right\}$$
$$+ \sum \left\{ e^{\beta'(-I_1'-I_1'-\kappa)} + e^{\beta'(-I_1'+I_1')} + e^{\beta'(I_1'-I_1')} + e^{\beta'(I_1'+I_1'-\kappa)} \right\} \qquad \cdots \ (18)$$

Solving the above relationships derives the following Equation (19) to Equation (21).

$$z_{\text{int}} = 2\left[ e^{\beta'\kappa} \cosh\{2\beta'(I_1'-3)\} + 1 \right] \qquad \cdots \ (19)$$

$$z_{rot} = \frac{2 \cdot I_M}{\beta} \cdot \frac{\sinh(\beta \cdot F \cdot a)}{\beta \cdot F \cdot a} \qquad \cdots \ (20)$$

$$z_{trans} = \exp\left\{ \lambda \cdot \Lambda \cdot \left( \frac{2\pi \cdot m}{\beta} \right)^{\frac{3}{2}} \right\} + 2\left[ e^{\beta'\kappa} \cosh\{2\beta'(I_1'-3)\} + 1 \right] \qquad \cdots \ (21)$$

These distribution functions are for a localized model as illustrated in Fig. 2, and the following Equation (22) to Equation (24) are obtained when expanded to a macro-model with N molecules.

$$\overline{Z}_{\text{int}} = \frac{z_{\text{int}}^N}{N!} \qquad \cdots \ (22)$$

$$\overline{Z}_{rot} = \frac{z_{rot}^N}{N!} \qquad \cdots (2\,3)$$

$$\overline{Z}_{trans} = \frac{z_{trans}^N}{N!} \qquad \cdots (2\,4)$$

Wherein: $\overline{Z}_{hit}$ represents a distribution function of intermolecular or intramolecular interaction expanded to N molecules, $\overline{Z}_{rot}$ represents a distribution function of molecular rotation expanded to N molecules, $\overline{Z}_{trans}$ represents a distribution function of molecular translation expanded to N molecules.

The relationships between the distribution functions and the thermodynamic state functions are expressed by the following Equation (25) to Equation (27).

$$U = -\left(\frac{\partial \ln \overline{\overline{Z}}_{int}}{\partial \beta'}\right)_V \qquad \cdots (2\,5)$$

$$S = k \cdot \ln \Omega(F) = \ln \overline{Z}_{rot} - \beta \cdot F \cdot a \qquad \cdots (2\,6)$$

$$pV = \frac{1}{\beta} \cdot \ln \overline{Z}_{trans} \qquad \cdots (2\,7)$$

Wherein $\beta$ represents 1/RT. When $T_g$ is the glass transition temperature, then $\beta'$ is expressed by:

$$\frac{1}{R(T - T_g)}$$

n represents a number of links between cross-link points in a statistical molecule chain, and E represents an intermolecular interaction energy coefficient. $\Omega$ (F) represents a number of states adopted by molecules, with F representing the force generated by a deformed molecule, $a$ represents a statistical molecule length, and k representing the Boltzmann constant. The above Equation (III) is derived by substituting a distribution function for N molecules into these equations. Consequently, a change in Helmholtz free energy due to deformation in rubber is expressed as a difference between a non-deformed state and a deformed state.

Each of the terms in above Equation (III) correspond to the respective Equations (IV) to (VI) above. Wherein, $I_1'$ considers a local interaction function $\lambda_{micro}$ and is defined by above Equation (VII).

In the elastic response performance prediction method of the present exemplary embodiment, the elastic response performance of a rubber product is predicted by employing a constitutive equation expressed by the Equation (III) to Equation (VII) derived as described above.

Moreover, in the present exemplary embodiment, the number of links n between cross-linked points in a statistical molecule chain is defined by the above Equation (I). $X_c$ represents the crystallization ratio, and is represented by above Equation (II) as a function of internal energy change $\Delta U = U_1 - U_0$ only in cases in which the subject material exhibits extension crystallization properties.

Wherein, $\Delta H$ when crystals melt is equivalent to the entropy of solution, and 6MJ/m$^3$ is employed as $\Delta H$ in cases of for natural rubber. Note that $X_c$ is taken as 0 in cases in which the subject material does not exhibit extension crystallization properties. Moreover, $X_c$ is never 1 or greater, since there can never be more than 100% crystallization due to extension.

In the a constitutive equation described above, appropriate application of the above Equation (I) and Equation (II) enables consideration to be made of the temperature and stress dependence even in cases in which stress-strain behavior analysis technology is applied at a micro-level in rubber with extension crystallization properties.

Moreover, by appropriate application of Equation (I) and Equation (II) to the Equations (III), (IV) and (VI), an internal energy term U is expressed as a negative value (exothermic) the larger the deformation of rubber with extension crystallization properties. Moreover, in a stretched molecule phenomenon there are limitations to the entropy S by molecular mobility between cross-linked points due to crystallization, and the value of n becomes smaller, and S increases, in proportion to the crystallization ratio. A constitutive equation is accordingly obtained expressing stretched molecules or non-linear behavior in regions where large deformation is observed due to extension crystallization.

Explanation follows regarding operation when prediction of deformation behavior in a rubber material is performed according to the first exemplary embodiment.

First, a 3D-model of a rubber product, such as a tire, is generated by the computer 50, and the 3D-model data is stored on the HDD.

Next, a user operates the input-output terminal to set the 3D-model to be subjected to analysis. The user then operates the input-output terminal to set analysis conditions for analysis processing. The analysis condition setting includes framework conditions of the rubber portion of the 3D-model, and setting framework conditions for filler portions. As framework conditions of the rubber portion of the 3D-model, the constitutive equation of the above Equations (I) to (VII) is set (when the rubber exhibits extension crystallization), or the constitutive equation of Equations (I) and (III) to (VII) is set (when the rubber does not exhibit extension crystallization).

The computer 50 then reads in the 3D-model data of the 3D-model set as the subject of analysis from the HDD, and applies the set analysis conditions as the framework conditions of the rubber portion and the filler portion of the 3D-model expressed by the read-in 3D-model data, so as to reconstruct the 3D-model data.

Next, the computer 50 employs the reconstructed 3D-model data to analyze with a finite element method the strain and the internal stress distribution in the 3D-model, and the stress values of the 3D-model as a whole resulting from vehicle load bearing on the tire, internal pressure of the tire, rolling of the tire etc. when the 3D-model is changed under the framework conditions that have been set.

The computer 50 then displays on a display the strain state and the internal stress distribution of the 3D-model, and the stress values of the 3D-model as a whole that have been derived by analysis, and then ends processing.

Then the constitutive equation described above, this being the Equations (I) and (III) to (VII), are employed and prediction of the temperature dependence of the stress-strain curve of the rubber is performed, and the temperature dependence of the stress-strain curve is also measured using the cross-linked styrene butadiene rubber. A comparison is then performed between the prediction values (solid line) by the prediction described above, and the measurement values (plots). A comparison result is illustrated in Fig. 3. It can be said that there is a good match shown between the curves obtained with the Equations (I) and (III) to (VII), particularly in low extension ratio regions, and the measurement value plots.

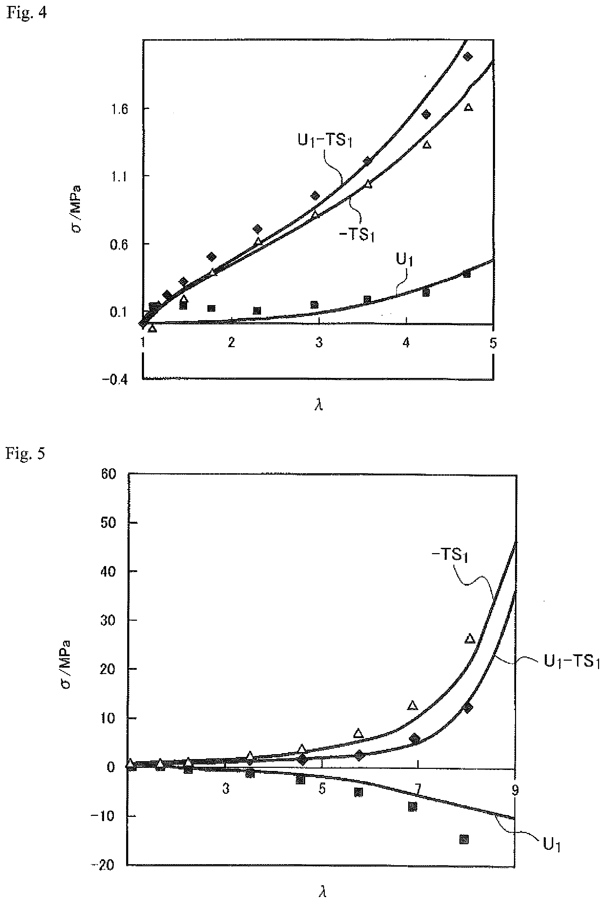

Moreover, cross-linked styrene butadiene rubber is employed, measurement of the stress due to energy elasticity and the stress due to entropy elasticity for a stress-strain curve at room temperature is performed, and computation is also performed of the corresponded portion ($U_1 - TS_1$) of the Equations (III), (IV) in the above constitutive equation. A comparison is performed between the above measurement value (plots) and computed values (solid lines), Comparison results are illustrated in Fig. 4. It can be seen that there is a good match between the curves obtained by the corresponded portion of the Equations (III), (IV) and the plots of the measurement values.

Moreover cross-linked natural rubber is employed, measurement is performed of the stress due to energy elasticity and the stress due to entropy elasticity for a stress-strain curve at room temperature, and the Equations (I) and (II) are also applied and computation performed of the corresponded portion ($U_1 - TS_1$) of the Equations (III), (IV) in the above constitutive equation. A comparison is made between the above measurement values (plots) and computed values (solid lines), Comparison results are illustrated in Fig. 5. It can be seen that there is a good match between the curves obtained by the corresponded portion of the Equations (III), (IV) and the plots of the measurement values. Since natural rubber exhibits extension crystallization, there is a tendency for the energy elasticity to become minus when the extension ratio is 3 or greater. Since such a phenomenon is also well represented, it can be said that good modeling can also be achieved even for extension crystallization.

As explained above, according to a rubber product elastic response performance prediction apparatus according to the first exemplary embodiment, a crystallization ratio expressing the extension crystallization at high strain in a rubber component exhibiting extension crystallization properties, such as NR, is incorporated into a constitutive equation expressing temperature and strain dependence of strain energy, so as to predict the elastic response performance of a rubber product. The elastic response performance can accordingly be predicted with good precision even if the rubber product exhibits extension crystallization properties.

Moreover, when a filler based rubber is observed at the micro-observation level, in a diffuse deformation region of a rubber product, a constitutive equation is employed that expresses temperature and strain dependence of strain energy employing a local interaction function $\lambda_{micro}$ incorporated with the effects of adjacent molecules at low strain, so as to predict the elastic response performance of a rubber product. Accordingly, elastic response performance in low temperature conditions, particularly in a low extension ratio region, can be predicted with good precision.

A microstructure model is defined, and errors in portions of interaction as viewed in micro, in particular extension crystallization properties of natural rubber in high deformation regions, are incorporated, enabling the divergence from observed values to be reduced. The elastic response performance of a rubber product containing natural rubber can accordingly be predicted with good precision.

Moreover, a feature is that the energy elasticity is considered in addition to conventional consideration of the contribution of entropy elasticity representing rubber elasticity. The energy elasticity is considered in a similar manner to the strain energy function proposed in Japanese Patent No. 4299735, however a microstructure model thereof was not clearly defined. Hence errors in the portions of interaction as viewed in micro, and in particular the contribution of energy elasticity in low deformation regions, and the phenomenon called extension crystallization where unable to be represented. In the present invention there is a clear microstructure model for statistical thermodynamic computation, and by deriving a constitutive equation based on this model, a universal constitutive equation for rubber elasticity is derived that does not depend on the deformation region or the type of rubber,

With a conventional rubber constitutive equation it was not possible to express the thermodynamic behavior of the rubber, as is illustrated in Fig. 4 and Fig. 5. In the present invention, a constitutive equation expressing the thermodynamic behavior of rubber such as illustrated in Fig. 4 and Fig. 5 is introduced into a conventional computational simulation. It is accordingly possible to perform predictive computation in a state reflecting the thermodynamic behavior of raw materials actually employed.

Moreover, the temperature dependence of a stress-strain curve expressed by employing the constitutive equation obtained from statistical thermodynamic computation has been confirmed to have a good match with results from measurements including those for natural rubber that exhibits extension crystallization properties. Thus by introducing the proposed constitutive equation into a finite element method stress-strain curve, it is possible to represent the rubber stress-strain behavior of rubber at the nanoscale for both rubbers with extension crystallization properties and rubbers without crystallization properties, including elasticity contributions, viscosity contributions, and plasticity contributions therein.

Explanation next follows regarding a second exemplary embodiment.

The second exemplary embodiment differs from the first exemplary embodiment regarding the prediction of elastic response performance of a rubber product in the point that a constitutive equation is employed that expresses the temperature and strain dependence of an elastic modulus of a rubber product.

In an elastic response performance prediction method of the second exemplary embodiment a constitutive equation expressed by above Equation (X), which is derived similarly to that of the first exemplary embodiment, is applied to a deformation behavior analysis technique for rubber at the micro-level to predict the elastic response performance of a rubber product.

Each of the terms in the above Equation (X) are expressed by the above Equations (XI), (XII) and (XIII). Wherein, $I_1$' is expressed by employing a local interaction function $\lambda_{micro}$ and is expressed by Equation (XIV).

In the present exemplary embodiment, the number of links n between cross-linked points in a statistical molecule chain is defined using the above Equations (VIII) and (IX), so as to represent extension crystallization. Thus temperature and strain dependence can be considered even in cases in which a deformation behavior analysis technique is applied at the micro-level to a rubber with extension crystallization properties.

Note that since other configuration and operation of the elastic response performance prediction apparatus according to a second exemplary embodiment of the present invention are similar to those of the first exemplary embodiment, explanation is omitted thereof.

Thus according to the rubber product elastic response performance prediction apparatus according to the second exemplary embodiment, a crystallization ratio expressing extension crystallization at high strain in a rubber component exhibiting extension crystallization properties, such as NR, is incorporated into a constitutive equation expressing the temperature and strain dependence of an elastic modulus, so as to predict the elastic response performance of a rubber product. The elastic response performance can accordingly be predicted with good precision even if the rubber product exhibits extension crystallization properties.

Moreover, when a filler based rubber is observed at the micro-observation level, in a diffuse deformation region of a rubber product, a constitutive equation is employed that expresses temperature and strain dependence of an elastic modulus employing a local interaction function $\lambda_{micro}$ incorporated with the effects of adjacent molecules at low strain, so as to predict the elastic response performance of a rubber product. Accordingly, elastic response performance in low temperature conditions, particularly in a low extension ratio region, can be predicted with good precision.

A microstructure model is defined, and errors in portions of interaction as viewed in micro, in particular extension crystallization properties of natural rubber in high deformation regions, are incorporated, enabling the divergence from observed values to be reduced. The elastic response performance of a rubber product containing natural rubber can accordingly be predicted with good precision.

**Claims**

1. A computer-implemented elastic response performance prediction method that predicts an elastic response performance expressing deformation behavior of a rubber product, the elastic response performance prediction method comprising:

generating a model of the rubber product,
operating an input-output terminal to set a constitutive equation as a framework condition of the rubber product, applying the framework condition to the model of the rubber product, and reconstructing model data of the model, deforming the model under the framework condition and predicting the elastic response performance of the rubber product, wherein the constitutive equation is a constitutive equation that expresses temperature and strain dependence of strain energy or of an elastic modulus of the rubber product, and that incorporates a number of links between cross-linked points in a statistical molecule chain which is expressed using a parameter representing extension crystallization, wherein:

a number of links n between the cross-linked points in the statistical molecule chain is expressed by the following Equation (I):

$$n = \alpha \cdot \left(1 - X_c\right) \cdot \exp\left(-\varepsilon \cdot \beta\right) \qquad \cdots \quad (\,I\,)$$

wherein $\alpha$ represents a frequency factor of statistical segment motion, $\varepsilon$ represents an activation energy of statistical segment motion, $\beta = 1/RT_g$ wherein R is a gas constant and $T_g$ is a glass transition temperature, $X_c$ represents a crystallization ratio as a parameter expressing the extension crystallization, and $X_c$ is expressed by the following Equation (II) when a material of the rubber product exhibits extension crystallization properties:

$$X_c = \left(\frac{U_1 - U_0}{\Delta H_0}\right) = \left(\frac{\Delta U}{\Delta H_0}\right) \qquad \cdots \quad (\,II\,)$$

wherein $U_0$ represents internal energy in a non-deformed state, $U_1$ represents internal energy in a deformed state, and $\Delta H_0$ represents entropy of solution when crystals melt.

2. The elastic response performance prediction method of claim 1, wherein the crystallization ratio $X_c$ is set at 0 when the material of the rubber product does not exhibit extension crystallization properties.

3. The elastic response performance prediction method of claim 1 or claim 2, wherein the constitutive equation is the following Equation (III):

$$\Delta A = \left(U_1 - TS_1\right) + p\left(V_1 - V_0\right) - \left(U_0 - TS_0\right) \qquad \cdots \quad (III)$$

wherein A represents Helmholtz free energy, $U_0$ represents internal energy in a non-deformed state, $U_1$ represents internal energy in a deformed state, p represents pressure, $V_0$ represents volume in a non-deformed state, $V_1$ represents volume in a deformed state, T represents absolute temperature, $S_0$ represents entropy in a non-deformed state, and $S_1$ represents entropy in a deformed state, with each of the terms of Equation (III) expressed by the following Equations (IV) to (VI):

$$U_1 - T \cdot S_1 = \frac{e^{\beta' \cdot \kappa} \left\{ \kappa \cosh\left(2\beta'\left(I_1'-3\right)\right) + 2\left(I_1'-3\right) \cdot \sinh\left(2\beta'\left(I_1'-3\right)\right) \right\}}{e^{\beta' \cdot \kappa} \cosh\left(2\beta'\left(I_1'-3\right)\right) + 1}$$

$$- \frac{N}{\beta} \cdot \left\{ \frac{1}{2} I_1 + \frac{3}{100n}\left(3I_1^2 - 4I_2\right) + \frac{99}{12250n}\left(5I_1^3 - 12I_1 I_2\right) \right\}$$

$$\cdots \text{(IV)}$$

$$p\left(V_1 - V_0\right) = B \cdot \left(V_1 - V_0\right) = B\left(I_3^{\frac{1}{2}} - 1\right)^2$$

$$- \frac{1}{\beta'}\left\{\ln\left[1 + e^{\beta' \cdot \kappa} \cosh\left(2\beta'\left(I_1'-3\right)\right)\right] - \ln\left[1 + e^{\beta' \kappa}\right]\right\}$$

$$\cdots \text{(V)}$$

$$U_0 - T \cdot S_0 = \frac{\kappa \cdot e^{\beta' \cdot \kappa}}{e^{\beta' \cdot \kappa} + 1} + \frac{N}{\beta}\left(\frac{3}{2} + \frac{45}{100n} + \frac{2673}{12250n^2}\right) \quad \cdots \text{(VI)}$$

wherein: $I_1$, $I_2$, and $I_3$ are expressed as functions of three extension ratios of deformation $\lambda_1$, $\lambda_2$ and $\lambda_3$ in xyz directions in three dimensional axes of rubber by $I_1 = \lambda_1^2 + \lambda_2^2 + \lambda_3^2$, $I_2 = \lambda_1^2 \cdot \lambda_2^2 + \lambda_2^2 \cdot \lambda_3^2 + \lambda_3^2 \cdot \lambda_1^2$, and $I_3 = \lambda_1^2 \cdot \lambda_2^2 \cdot \lambda_3^2$, n represents the number of links between the cross-linked points in the statistical molecule chain, $\kappa$ expresses an intermolecular interaction energy coefficient, $\beta = 1/RT$ and $\beta' = 1/R (T - T_g)$ wherein R is a gas constant and $T_g$ is a glass transition temperature, B represents bulk modulus, N represents number of molecules, and $I_1'$ is expressed using a local interaction function $\lambda_{micro}$ as a parameter expressing the intermolecular interaction by the following Equation (VII):

$$I_1' = \lambda_{micro}^2\left(\lambda_1^2 + \lambda_2^2 + \lambda_3^2\right) = \lambda_{micro}^2 \cdot I_1 \quad \cdots \text{(VII)}$$

4. The elastic response performance prediction method of claim 1 or claim 2, wherein the constitutive equation is the following Equation (X):

$$G = \frac{\partial W}{\partial I_1} = \frac{\partial A}{\partial I_1} = \frac{\partial U}{\partial I_1} - T \cdot \frac{\partial S}{\partial I_1} + \frac{\partial pV}{\partial I_1} \quad \cdots \text{(X)}$$

wherein G represents a shear elastic modulus, W represents a strain energy coefficient, A represents Helmholtz free energy, U represents internal energy, T represents absolute temperature, S represents entropy, and $I_1$ is expressed as a function of three extension ratios of deformation $\lambda_1$, $\lambda_2$ and $\lambda_3$ in xyz directions in three dimensional axes of rubber by $I_1 = \lambda_1^2 + \lambda_2^2 + \lambda_3^2$, with each of the terms of Equation (X) respectively expressed by the following Equation (XI), Equation (XII), and Equation (XIII):

$$\frac{\partial U}{\partial I_1}$$

$$= \frac{-e^{\beta'\kappa}\left\{2e^{\beta'\kappa}\sinh\left(2\beta'\left(I_1'-3\right)\right)\cosh\left(2\beta'\left(I_1'-3\right)\right) + 2\left(\beta'\kappa + 1\right)\sinh\left(2\beta'\left(I_1'-3\right)\right) + 4\beta'\left(I_1'-3\right)\cosh\left(2\beta'\left(I_1'-3\right)\right) + 4\beta'\left(I_1'-3\right)e^{\beta'\kappa}\right\}}{\left\{e^{\beta'\kappa} \cdot \cosh\left(2\beta'\left(I_1'-3\right)\right) + 1\right\}^2}$$

$$\cdots \text{(XI)}$$

$$\frac{\partial S}{\partial I_1} = -vR\left[\frac{1}{2} + \frac{3}{50n}\left(3I_1 - \frac{2}{\lambda}\right) + \frac{297}{6125n^2}\left(5I_1^{\ 2} - 4I_2 - 4\frac{I_1}{\lambda}\right)\right] \qquad \cdots \ (\text{XII})$$

$$\frac{\partial pV}{\partial I_1} = \frac{2 \cdot e^{\beta'\kappa} \cdot \cosh(2\beta'(I_1' - 3))}{e^{\beta'\kappa} \cdot \cosh(2\beta'(I_1' - 3)) + 1} \qquad \cdots \ (\text{XIII})$$

wherein $\kappa$ expresses an intermolecular interaction energy coefficient, n represents the number of links between the cross-linked points in the statistical molecule chain, v represents a cross-link density, $\lambda$ represents an extension ratio or compression ratio, $\beta'$ = 1/R (T - $T_g$) wherein R is a gas constant and $T_g$ is a glass transition temperature, P represents pressure, V represents volume, $I_2$ is represented by $I_2 = \lambda_1^2 \cdot \lambda_2^2 + \lambda_2^2 \cdot \lambda_3^2 + \lambda_3^2 \cdot \lambda_1^2$, and $I_1'$ is expressed using a local interaction function $\lambda_{micro}$ as a parameter expressing the intermolecular interaction by the following Equation (XIV):

$$I_1' = \lambda_{micro}^{\ \ 2}\left(\lambda_1^{\ 2} + \lambda_2^{\ 2} + \lambda_3^{\ 2}\right) = \lambda_{micro}^{\ \ 2} \cdot I_1 \qquad \cdots \ (\text{XIV})$$

5. The elastic response performance prediction method of any one of claim 1 to claim 4, wherein the elastic response performance expressing deformation behavior of the rubber product is predicted using a finite element analysis method.

6. A rubber product design method comprising designing a rubber product by employing the elastic response performance prediction method of any one of claim 1 to claim 5.

7. An elastic response performance prediction apparatus (50) that predicts elastic response performance expressing deformation behavior of a rubber product, the elastic response performance prediction apparatus (50):

    being capable of generating a model of the rubber product,
    having an input-output terminal capable of being operated to set a constitutive equation as a framework condition of the rubber product,
    being capable of applying the framework condition to the model of the rubber product, and reconstructing model data of the model,
    being capable of deforming the model under the framework condition and predicting the elastic response performance of the rubber product by employing a constitutive equation that expresses temperature and strain dependence of strain energy or of an elastic modulus of the rubber product, and that incorporates a number of links between cross-linked points in a statistical molecule chain which is expressed using a parameter representing extension crystallization, wherein:

    a number of links n between the cross-linked points in the statistical molecule chain is expressed by the following Equation (I):

$$n = \alpha \cdot \left(1 - X_c\right) \cdot \exp\left(-\varepsilon \cdot \beta\right) \qquad \cdots \ (\text{I})$$

    wherein $\alpha$ represents a frequency factor of statistical segment motion, $\varepsilon$ represents an activation energy of statistical segment motion, $\beta$ = 1/$RT_g$ wherein R is a gas constant and $T_g$ is a glass transition temperature, $X_c$ represents a crystallization ratio as a parameter expressing the extension crystallization, and $X_c$ is expressed by the following Equation (II) when a material of the rubber product exhibits extension crystallization properties:

$$X_c = \left( \frac{U_1 - U_0}{\Delta H_0} \right) = \left( \frac{\Delta U}{\Delta H_0} \right) \qquad \cdot \; \cdot \; \cdot \; (\text{II})$$

wherein $U_0$ represents internal energy in a non-deformed state, $U_1$ represents internal energy in a deformed state, and $\Delta H_0$ represents entropy of solution when crystals melt.

**Patentansprüche**

1. Computerimplementiertes Verfahren zur Vorhersage einer elastischen Ansprechleistung, das eine elastische Ansprechleistung vorhersagt, die das Verformungsverhalten eines Kautschukprodukts ausdrückt, das Verfahren zur Vorhersage einer elastischen Ansprechleistung umfassend:

   Erzeugen eines Modells des Kautschukprodukts,
   Betreiben eines Ein-/Ausgangsendgeräts zum Einstellen einer Zustandsgleichung als eine Rahmenbedingung des Kautschukprodukts,
   Anwenden der Rahmenbedingung auf das Modell des Kautschukprodukts und Rekonstruieren von Modelldaten des Modells,
   Verformen des Modells unter der Rahmenbedingung und Vorhersagen der elastischen Ansprechleistung des Kautschukprodukts, wobei die Zustandsgleichung eine Zustandsgleichung ist, die Temperatur- und Formänderungsabhängigkeit von Formänderungsenergie oder von einem Elastizitätsmodul des Kautschukprodukts ausdrückt und die eine Anzahl von Verknüpfungen zwischen quervernetzten Punkten in einer statistischen Molekülkette inkorporiert, wenn sie unter Verwendung eines Parameters, der Ausdehnungskristallisierung repräsentiert, ausgedrückt wird, wobei:

   eine Anzahl von Verknüpfungen n zwischen den quervernetzten Punkten in der statistischen Molekülkette durch die folgende Gleichung (I) ausgedrückt wird:

$$n = \alpha \cdot \left( 1 - X_c \right) \cdot \exp\!\left( -\varepsilon \cdot \beta \right) \qquad \cdot \; \cdot \; \cdot \; (\text{I})$$

   wobei $\alpha$ einen Frequenzfaktor von statistischer Segmentbewegung repräsentiert, $\varepsilon$ eine Aktivierungsenergie von statistischer Segmentbewegung repräsentiert, $\beta = 1/RT_g$ ist, wobei R eine Gaskonstante ist und $T_g$ eine Glasübergangstemperatur ist, $X_c$ ein Kristallisierungsverhältnis als einen Parameter repräsentiert, der die Ausdehnungskristallisierung ausdrückt, und $X_c$ durch die folgende Gleichung (II) ausgedrückt wird, wenn ein Material des Kautschukprodukts Ausdehnungskristallisierungseigenschaften aufzeigt:

$$X_c = \left( \frac{U_1 - U_0}{\Delta H_0} \right) = \left( \frac{\Delta U}{\Delta H_0} \right) \qquad \cdot \; \cdot \; \cdot \; (\text{II})$$

   wobei $U_0$ interne Energie in einem nicht verformten Zustand repräsentiert, $U_1$ interne Energie in einem verformten Zustand repräsentiert und $\Delta H_0$ Lösungsentropie beim Schmelzen von Kristallen repräsentiert.

2. Verfahren zur Vorhersage einer elastischen Ansprechleistung nach Anspruch 1, wobei das Kristallisierungsverhältnis $X_c$ auf 0 gestellt wird, wenn das Material des Kautschukprodukts keine Ausdehnungskristallisierungseigenschaften aufzeigt.

3. Verfahren zur Vorhersage einer elastischen Ansprechleistung nach Anspruch 1 oder Anspruch 2, wobei die Zustandsgleichung die folgende Gleichung (III) ist:

$$\Delta A = \left(U_1 - TS_1\right) + p\left(V_1 - V_0\right) - \left(U_0 - TS_0\right) \qquad \cdots \quad (\text{III})$$

wobei A freie Helmholtz-Energie repräsentiert, $U_0$ interne Energie in einem nicht verformten Zustand repräsentiert, $U_1$ interne Energie in einem verformten Zustand repräsentiert, p Druck repräsentiert, $V_0$ Volumen in einem nicht verformten Zustand repräsentiert, $V_1$ Volumen in einem verformten Zustand repräsentiert, T absolute Temperatur repräsentiert, $S_0$ Entropie in einem nicht verformten Zustand repräsentiert und $S_1$ Entropie in einem verformten Zustand repräsentiert, wobei jeder der Terme von Gleichung (II) durch die folgenden Gleichungen (IV) bis (VI) ausgedrückt wird:

$$U_1 - T \cdot S_1 = \frac{e^{\beta'\cdot\kappa}\{\kappa\cosh(2\beta'(I_1'-3)) + 2(I_1'-3)\cdot\sinh(2\beta'(I_1'-3))\}}{e^{\beta'\cdot\kappa}\cosh(2\beta'(I_1'-3)) + 1}$$
$$- \frac{N}{\beta}\cdot\left\{\frac{1}{2}I_1 + \frac{3}{100n}\left(3I_1^2 - 4I_2\right) + \frac{99}{12250n}\left(5I_1^3 - 12I_1I_2\right)\right\} \qquad \cdots \quad (\text{IV})$$

$$p\left(V_1 - V_0\right) = B\cdot\left(V_1 - V_0\right) = B\left(I_3^{\frac{1}{2}} - 1\right)^2$$
$$- \frac{1}{\beta'}\left\{\ln\left[1 + e^{\beta'\cdot\kappa}\cosh(2\beta'(I_1'-3))\right] - \ln\left[1 + e^{\beta'\cdot\kappa}\right]\right\} \qquad \cdots \quad (\text{V})$$

$$U_0 - T \cdot S_0 = \frac{\kappa\cdot e^{\beta'\cdot\kappa}}{e^{\beta'\cdot\kappa} + 1} + \frac{N}{\beta}\left(\frac{3}{2} + \frac{45}{100n} + \frac{2673}{12250n^2}\right) \qquad \cdots \quad (\text{VI})$$

wobei: $I_1$, $I_2$ und $I_3$ als Funktionen von drei Ausdehnungsverhältnissen der Deformation $\lambda_1$, $\lambda_2$ und $\lambda_3$ in den Richtungen xyz in drei Dimensionsachsen von Kautschuk durch $I_1 = \lambda_1^2 + \lambda_2^2 + \lambda_3^2$, $I_2 = \lambda_1^2\cdot\lambda_2^2 + \lambda_2^2\cdot\lambda_3^2 + \lambda_3^2\cdot\lambda_1^2$ und $I_3 = \lambda_1^2\cdot\lambda_2^2\cdot\lambda_3^2$ ausgedrückt werden, n die Anzahl von Verknüpfungen zwischen den quervernetzten Punkten in der statistischen Molekülkette repräsentiert, $\kappa$ einen Energiekoeffizienten der intermolekularen Wechselwirkung repräsentiert, $\beta = 1/RT$ und $\beta' = 1/R(T-T_g)$ sind, wobei R eine Gaskonstante ist und $T_g$ eine Glasübergangstemperatur ist, B den Kompressionsmodul repräsentiert, N die Anzahl von Molekülen repräsentiert und $I_1'$ unter Verwendung einer lokalen Interaktionsfunktion $\lambda_{micro}$ als ein Parameter, der die intermolekulare Wechselwirkung durch die folgende Gleichung (VII) ausdrückt, ausgedrückt wird:

$$I_1' = \lambda_{micro}^2\left(\lambda_1^2 + \lambda_2^2 + \lambda_3^2\right) = \lambda_{micro}^2\cdot I_1 \qquad \cdots \quad (\text{VII})$$

**4.** Verfahren zur Vorhersage einer elastischen Ansprechleistung nach Anspruch 1 oder Anspruch 2, wobei die Zustandsgleichung die folgende Gleichung (X) ist:

$$G = \frac{\partial W}{\partial I_1} = \frac{\partial A}{\partial I_1} = \frac{\partial U}{\partial I_1} - T\cdot\frac{\partial S}{\partial I_1} + \frac{\partial pV}{\partial I_1} \qquad \cdots \quad (\text{X})$$

wobei G einen Schubelastizitätsmodul repräsentiert, W einen Formänderungsenergiekoeffizienten repräsentiert, A freie Helmholtz-Energie repräsentiert, U interne Energie repräsentiert, T absolute Temperatur repräsentiert, S Entropie repräsentiert und $I_1$ als eine Funktion von drei Ausdehnungsverhältnissen der Deformation $\lambda_1$, $\lambda_2$ und $\lambda_3$ in den Richtungen xyz in drei Dimensionsachsen von Kautschuk durch $I_1 = \lambda_1^2 + \lambda_2^2 + \lambda_3^2$ ausgedrückt wird, wobei jeder der Terme der Gleichung (X) jeweils durch die folgende Gleichung (XI), Gleichung (XII) und Gleichung (XIII) ausgedrückt wird:

$$\frac{\partial U}{\partial I_1} = \frac{- e^{\beta'\kappa}\left\{2e^{\beta'\kappa}\sinh(2\beta'(I_1'-3))\cosh(2\beta'(I_1'-3)) + 2(\beta'\kappa+1)\sinh(2\beta'(I_1'-3)) + 4\beta'(I_1'-3)\cosh(2\beta'(I_1'-3)) + 4\beta'(I_1'-3)e^{\beta'\kappa}\right\}}{\left\{e^{\beta'\kappa}\cdot\cosh(2\beta'(I_1'-3))+1\right\}^2} \quad \cdots \quad (X\,I)$$

$$\frac{\partial S}{\partial I_1} = -vR\left[\frac{1}{2} + \frac{3}{50n}\left(3I_1 - \frac{2}{\lambda}\right) + \frac{297}{6125n^2}\left(5I_1^2 - 4I_2 - 4\frac{I_1}{\lambda}\right)\right] \quad \cdots \quad (X\,II)$$

$$\frac{\partial pV}{\partial I_1} = \frac{2\cdot e^{\beta'\kappa}\cdot\cosh(2\beta'(I_1'-3))}{e^{\beta'\kappa}\cdot\cosh(2\beta'(I_1'-3))+1} \quad \cdots \quad (X\,III)$$

wobei $\kappa$ einen Energiekoeffizienten der intermolekularen Wechselwirkung repräsentiert, n die Anzahl von Verknüpfungen zwischen den quervernetzten Punkten in der statistischen Molekülkette repräsentiert, v eine Quervernetzungsdichte repräsentiert, $\lambda$ ein Ausdehnungsverhältnis oder Kompressionsverhältnis repräsentiert, $\beta' = 1/R(T-T_g)$ ist, wobei R eine Gaskonstante ist und $T_g$ eine Glasübergangstemperatur ist, P Druck repräsentiert, V Volumen repräsentiert, $I_2$ durch $I_2 = \lambda_1^2\cdot\lambda_2^2 + \lambda_2^2\cdot\lambda_3^2 + \lambda_3^2\cdot\lambda_1^2$ repräsentiert wird und $I_1'$ unter Verwendung einer lokalen Interaktionsfunktion $\lambda_{micro}$ als ein Parameter, der die intermolekulare Wechselwirkung durch die folgende Gleichung (XIV) ausdrückt, ausgedrückt wird:

$$I_1' = \lambda_{micro}^2\left(\lambda_1^2 + \lambda_2^2 + \lambda_3^2\right) = \lambda_{micro}^2\cdot I_1 \quad \cdots \quad (X\,IV)$$

5. Verfahren zur Vorhersage einer elastischen Ansprechleistung nach einem der Ansprüche 1 bis 4, wobei die elastische Ansprechleistung, die Verformungsverhalten des Kautschukprodukts ausdrückt, unter Verwendung eines Finite-Elemente-Analyseverfahrens vorhergesagt wird.

6. Kautschukprodukt-Entwurfsverfahren, umfassend Entwerfen eines Kautschukprodukts durch Einsetzen des Verfahrens zur Vorhersage einer elastischen Ansprechleistung nach einem der Ansprüche 1 bis 5.

7. Vorrichtung zur Vorhersage einer elastischen Ansprechleistung (50), die eine elastische Ansprechleistung vorhersagt, die das Verformungsverhalten eines Kautschukprodukts ausdrückt, wobei die Vorrichtung zur Vorhersage einer elastischen Ansprechleistung (50):

imstande ist zum Erzeugen eines Modells des Kautschukprodukts,
ein Ein-/Ausgangsendgerät aufweist, das imstande ist, zum Einstellen einer Zustandsgleichung als eine Rahmenbedingung des Kautschukprodukts betrieben zu werden,
imstande ist zum Anwenden der Rahmenbedingung auf das Modell des Kautschukprodukts und zum Rekonstruieren von Modelldaten des Modells,
imstande ist zum Verformen des Modells unter der Rahmenbedingung und zum Vorhersagen der elastischen Ansprechleistung des Kautschukprodukts durch Einsetzen einer Zustandsgleichung, die Temperatur- und Formänderungsabhängigkeit von Formänderungsenergie oder von einem Elastizitätsmodul des Kautschukprodukts ausdrückt und die eine Anzahl von Verknüpfungen zwischen quervernetzten Punkten in einer statistischen Molekülkette inkorporiert, die unter Verwendung eines Parameters, der Ausdehnungskristallisierung repräsentiert, ausgedrückt wird, wobei:

eine Anzahl von Verknüpfungen n zwischen den quervernetzten Punkten in der statistischen Molekülkette durch die folgende Gleichung (I) ausgedrückt wird:

$$n = \alpha \cdot (1 - X_c) \cdot \exp(-\varepsilon \cdot \beta) \qquad \cdots \quad (\mathrm{I})$$

wobei $\alpha$ einen Frequenzfaktor von statistischer Segmentbewegung repräsentiert, $\varepsilon$ eine Aktivierungsenergie von statistischer Segmentbewegung repräsentiert, $\beta = 1/RT_g$ ist, wobei R eine Gaskonstante ist und $T_g$ eine Glasübergangstemperatur ist, $X_c$ ein Kristallisierungsverhältnis als einen Parameter repräsentiert, der die Ausdehnungskristallisierung ausdrückt, und $X_c$ durch die folgende Gleichung (II) ausgedrückt wird, wenn ein Material des Kautschukprodukts Ausdehnungskristallisierungseigenschaften aufzeigt:

$$X_c = \left(\frac{U_1 - U_0}{\Delta H_0}\right) = \left(\frac{\Delta U}{\Delta H_0}\right) \qquad \cdots \quad (\mathrm{II})$$

wobei $U_0$ interne Energie in einem nicht verformten Zustand repräsentiert, $U_1$ interne Energie in einem verformten Zustand repräsentiert und $\Delta H_0$ Lösungsentropie beim Schmelzen von Kristallen repräsentiert.

## Revendications

1. Procédé de prédiction de performances de réponse élastique implémenté sur ordinateur qui prédit une performance de réponse élastique exprimant un comportement de déformation d'un produit de caoutchouc, le procédé de prédiction de performance de réponse élastique comprenant :

la génération d'un modèle du produit de caoutchouc,
l'actionnement d'un terminal d'entrée-sortie permettant d'établir une équation constitutive comme condition cadre du produit de caoutchouc,
l'application de la condition cadre au modèle du produit de caoutchouc, et la reconstitution des données de modèle du modèle,
la déformation du modèle dans la condition cadre et la prédiction de la performance de réponse élastique du produit de caoutchouc, dans lequel l'équation constitutive est une équation constitutive qui exprime la dépendance de la température et de la contrainte de l'énergie de contrainte ou d'un module d'élasticité du produit de caoutchouc, et qui incorpore un nombre de liens entre des points réticulés dans une chaîne de molécule statistique qui est exprimée en utilisant un paramètre représentant une cristallisation d'extension, dans lequel :

un certain nombre de liens n entre les points réticulés dans la chaîne de molécule statistique est exprimé par l'Équation (I) suivante :

$$n = \alpha \cdot (1 - X_c) \cdot \exp(-\varepsilon \cdot \beta) \qquad \cdots \quad (\mathrm{I})$$

dans laquelle $\alpha$ représente un facteur de fréquence d'un mouvement de segment statistique, $\varepsilon$ représente une énergie d'activation d'un mouvement de segment statistique, $\beta = 1/RT_g$ dans laquelle R est une constante de gaz et $T_g$ est une température de transition vitreuse, $X_c$ représente un rapport de cristallisation comme paramètre exprimant la cristallisation d'extension, et $X_c$ est exprimée par l'Équation (II) suivante lorsqu'un matériau du produit de caoutchouc présente des propriétés de cristallisation d'extension :

$$X_c = \left(\frac{U_1 - U_0}{\Delta H_0}\right) = \left(\frac{\Delta U}{\Delta H_0}\right) \qquad \cdots \quad (\text{II})$$

dans laquelle $U_0$ représente une énergie interne dans un état non-déformé, $U_1$ représente une énergie interne dans un état déformé, et $\Delta H_0$ représente une entropie de solution lorsque des cristaux fondent.

2. Procédé de prédiction de performance de réponse élastique selon la revendication 1, dans lequel le rapport de cristallisation $X_c$ est établi à 0 lorsque le matériau du produit de caoutchouc ne présente pas de propriétés de cristallisation d'extension.

3. Procédé de prédiction de performance de réponse élastique selon la revendication 1 ou la revendication 2, dans lequel l'équation constitutive est l'Équation (III) suivante :

$$\Delta A = \left(U_1 - TS_1\right) + p\left(V_1 - V_0\right) - \left(U_0 - TS_0\right) \qquad \cdots \quad (\text{III})$$

dans laquelle A représente une énergie libre de Helmholz, $U_0$ représente une énergie interne dans un état non-déformé, $U_1$ représente une énergie interne dans un état déformé, p représente une pression, $V_0$ représente un volume dans un état non déformé, $V_1$ représente un volume dans un état déformé, T représente une température absolue, $S_0$ représente une entropie dans un état non-déformé, et $S_1$ représente une entropie dans un état déformé, chacun des termes de l'Équation (III) étant exprimé par les Équations (IV) à (VI) suivantes :

$$U_1 - T \cdot S_1 = \frac{e^{\beta' \cdot \kappa}\{\kappa \cosh(2\beta'(I_1'-3)) + 2(I_1'-3)\cdot \sinh(2\beta'(I_1'-3))\}}{e^{\beta' \cdot \kappa}\cosh(2\beta'(I_1'-3)) + 1}$$
$$- \frac{N}{\beta}\cdot\left\{\frac{1}{2}I_1 + \frac{3}{100n}\left(3I_1^2 - 4I_2\right) + \frac{99}{12250n}\left(5I_1^3 - 12I_1 I_2\right)\right\} \qquad \cdots \quad (\text{IV})$$

$$p(V_1 - V_0) = B \cdot (V_1 - V_0) = B\left(I_3^{\frac{1}{2}} - 1\right)^2$$
$$- \frac{1}{\beta'}\left\{\ln\left[1 + e^{\beta' \cdot \kappa}\cosh(2\beta'(I_1'-3))\right] - \ln\left[1 + e^{\beta' \cdot \kappa}\right]\right\} \qquad \cdots \quad (\text{V})$$

$$U_0 - T \cdot S_0 = \frac{\kappa \cdot e^{\beta' \cdot \kappa}}{e^{\beta' \cdot \kappa} + 1} + \frac{N}{\beta}\left(\frac{3}{2} + \frac{45}{100n} + \frac{2673}{12250n^2}\right) \qquad \cdots \quad (\text{VI})$$

dans laquelle : $I_1$, $I_2$ et $I_3$ sont exprimées en fonction de trois rapports d'extension de déformation $\lambda_1$, $\lambda_2$ et $\lambda_3$ dans des directions xyz dans trois axes dimensionnels du caoutchouc par $I = \lambda_1^2 + \lambda_2^2 + \lambda_3^2$, $I_2 = \lambda_1^2 \cdot \lambda_2^2 + \lambda_2^2 \cdot \lambda_3^2 + \lambda_3^2 \cdot \lambda_1^2$ et $I_3 = \lambda_1^2 \cdot \lambda_2^2 \cdot \lambda_3^2$, n représente le nombre de liens entre les points réticulés dans la chaine de molécules statistiques, $\kappa$ exprime un coefficient d'énergie d'interaction intermoléculaire, $\beta = 1/RT$ et $\beta' = 1/R(T - T_g)$, dans lequel R est une constante de gaz et Tg est une température de transition vitreuse, B représente un module de compression, N représente un nombre de molécules, et $I_1'$ est exprimé en utilisant une fonction d'interaction locale $\lambda_{micro}$ comme paramètre exprimant l'interaction intermoléculaire par l'Équation (VII) suivante :

$$I_1' = \lambda_{micro}{}^2\left(\lambda_1{}^2 + \lambda_2{}^2 + \lambda_3{}^2\right) = \lambda_{micro}{}^2 \cdot I_1 \qquad \cdots \quad (VII)$$

**4.** Procédé de prédiction de performance de réponse élastique selon la revendication 1 ou la revendication 2, dans lequel l'équation constitutive est l'Équation (X) suivante :

$$G = \frac{\partial W}{\partial I_1} = \frac{\partial A}{\partial I_1} = \frac{\partial U}{\partial I_1} - T \cdot \frac{\partial S}{\partial I_1} + \frac{\partial pV}{\partial I_1} \qquad \cdots \quad (X)$$

dans laquelle G représente un module élastique de cisaillement, W représente un coefficient d'énergie de contrainte, A représente une énergie libre de Helmholtz, U représente une énergie interne, T représente une température absolue, S représente une entropie, et $I_1$ est exprimé en fonction de trois rapports d'extension de déformation $\lambda_1$, $\lambda_2$ et $\lambda_3$ dans des directions xyz dans trois axes dimensionnels du caoutchouc par $I_1 = \lambda_1{}^2 + \lambda_2{}^2 + \lambda_3{}^2$, chacun des termes de l'Équation (X) étant respectivement exprimé par l'Équation (XI), l'Équation (XII) et l'Équation (XIII) suivantes :

$$\frac{\partial U}{\partial I_1}$$
$$= \frac{-e^{\beta'\kappa}\left\{2e^{\beta'\kappa}\sinh(2\beta'(I_1'-3))\cosh(2\beta'(I_1'-3)) + 2(\beta'\kappa+1)\sinh(2\beta'(I_1'-3)) + 4\beta'(I_1'-3)\cosh(2\beta'(I_1'-3)) + 4\beta'(I_1'-3)e^{\beta'\kappa}\right\}}{\left\{e^{\beta'\kappa}\cdot\cosh(2\beta'(I_1'-3)) + 1\right\}^2} \qquad \cdots \quad (XI)$$

$$\frac{\partial S}{\partial I_1} = -vR\left[\frac{1}{2} + \frac{3}{50n}\left(3I_1 - \frac{2}{\lambda}\right) + \frac{297}{6125n^2}\left(5I_1{}^2 - 4I_2 - 4\frac{I_1}{\lambda}\right)\right] \qquad \cdots \quad (XII)$$

$$\frac{\partial pV}{\partial I_1} = \frac{2 \cdot e^{\beta'\kappa} \cdot \cosh(2\beta'(I_1'-3))}{e^{\beta'\kappa} \cdot \cosh(2\beta'(I_1'-3)) + 1} \qquad \cdots \quad (XIII)$$

dans lesquelles $\kappa$ exprime un coefficient d'énergie d'interaction intermoléculaire, n représente le nombre de liens entre les points réticulés dans la chaîne de molécules statistiques, v représente une densité de réticulation, $\lambda$ représente un rapport d'extension ou un rapport de compression, $\beta' = 1/R\,(T - T_g)$, dans lequel R est une constante de gaz et $T_g$ est une température de transition vitreuse, P représente une pression, V représente un volume, $I_2$ est représentée par $I_2 = \lambda_1{}^2 \cdot \lambda_2{}^2 + \lambda_2{}^2 \cdot \lambda_3{}^2 + \lambda_3{}^2 \cdot \lambda_1{}^2$ et $I_1'$ est exprimée en utilisant une fonction d'interaction locale $\lambda_{micro}$ comme paramètre exprimant l'interaction intermoléculaire par l'Équation (XIV) suivante :

$$I_1' = \lambda_{micro}{}^2\left(\lambda_1{}^2 + \lambda_2{}^2 + \lambda_3{}^2\right) = \lambda_{micro}{}^2 \cdot I_1 \qquad \cdots \quad (XIV)$$

**5.** Procédé de prédiction de performances de réponse élastique selon l'une quelconque des revendications 1 à 4, dans lequel la performance de réponse élastique exprimant un comportement de déformation du produit de caoutchouc est prédite en utilisant un procédé d'analyse aux éléments finis.

**6.** Procédé de conception d'un produit de caoutchouc, comprenant la conception d'un produit de caoutchouc en employant le procédé de prédiction de performances de réponse élastique selon l'une quelconque des revendications 1 à 5.

7. Appareil de prédiction de performances de réponse élastique (50) qui prédit la performance de réponse élastique exprimant un comportement de déformation d'un produit de caoutchouc, l'appareil de prédiction de performance de réponse élastique (50) :

étant capable de générer un modèle du produit de caoutchouc,
présentant une borne d'entrée-sortie capable d'être actionnée afin d'établir une équation constitutive comme une condition cadre du produit de caoutchouc,
étant capable d'appliquer la condition cadre au modèle du produit de caoutchouc et de reconstituer les données du modèle,
étant capable de déformer le modèle dans la condition cadre et de prédire la performance de réponse élastique du produit de caoutchouc en employant une équation constitutive qui exprime la température et la dépendance de la contrainte de l'énergie de contrainte ou d'un module d'élasticité du produit de caoutchouc, et qui intègre un nombre de liens entre des points réticulés dans une chaine de molécule statistique qui est exprimé en utilisant un paramètre représentant une cristallisation par extension, dans lequel :

un certain nombre de liens n entre les points réticulés dans la chaîne de molécule statistique est exprimé par l'Équation (I) suivante :

$$n = \alpha \cdot \left(1 - X_c\right) \cdot \exp\left(-\varepsilon \cdot \beta\right) \qquad \ldots \text{ ( I )}$$

dans laquelle $\alpha$ représente un facteur de fréquence de mouvement de segment statistique, $\varepsilon$ représente une énergie d'activation du mouvement de segment statistique, $\beta = 1/RT_g$ dans laquelle R est une constante de gaz et $T_g$ est une température de transition vitreuse, $X_c$ représente un rapport de cristallisation comme un paramètre exprimant la cristallisation d'extension, et $X_c$ est exprimé par l'Équation (II) suivante lorsqu'un matériau du produit de caoutchouc présente des propriétés de cristallisation d'extension :

$$X_c = \left(\frac{U_1 - U_0}{\Delta H_0}\right) = \left(\frac{\Delta U}{\Delta H_0}\right) \qquad \ldots \text{ ( II )}$$

dans laquelle $U_0$ représente une énergie interne dans un état non-déformé, $U_1$ représente une énergie interne dans un état déformé, et $\Delta H_0$ représente une entropie de solution lorsque des cristaux fondent.

Fig. 1

Fig. 2

Fig. 3

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4299735 B

- JP 2007272416 A

### Non-patent literature cited in the description

- **E. ARRUDA et al.** A three-dimensional constitutive model for the large stretch behavior of rubber elastic materials. *Journal of the Mechanics and Physics of Solids,* 1993, vol. 41 (2), 389-412

- **Y. MIYAMOTO.** Crystallization and Melting of Polyisoprene Rubber under Uniaxial Deformation. *Journal of the Society of Rubber Industry,* 2004, vol. 77 (1), 24-29